# EUROPEAN PATENT SPECIFICATION

(11) **EP 2 579 849 B1**
(45) Date of publication and mention of the grant of the patent: **17.03.2021**
(21) Application number: 11724201.6
(22) Date of filing: 08.06.2011
(51) Int. Cl.: A61K 9/107, A61K 9/00, A61K 39/00

(54) **NOVEL PROCESS**
NEUES VERFAHREN
NOUVEAU PROCÉDÉ

(30) Priority: 10.06.2010 GB 201009673
(43) Date of publication of application: 17.04.2013
(73) Proprietor: GlaxoSmithKline Biologicals S.A., 1330 Rixensart (BE)
(72) Inventor: DE CUPERE, Vinciane, B-1330 Rixensart (BE); MANCUSO, Vincent, B-1330 Rixensart (BE)
(74) Representative: Thornley, Rachel Mary
(86) International application number: PCT/EP2011/059488
(87) International publication number: WO 2011/154443

(56) References cited:
- WO-A1-95/11700
- WO-A1-2007/062831
- WO-A2-2004/087204
- I. BURGAUD ET AL: "An improved high-pressure homogenizer for making fine emulsions on a small scale", INTERNATIONAL JOURNAL OF FOOD SCIENCE AND TECHNOLOGY, vol. 25, no. 1, 1 February 1990 (1990-02-01), pages 39-46, XP55008283, ISSN: 0950-5423, DOI: 10.1111/j.1365-2621.1990.tb01057.x
- PERRIER-CORNET J M ET AL: "Comparison of emulsification efficiency of protein-stabilized oil-in-water emulsions using jet, high pressure and colloid mill homogenization", JOURNAL OF FOOD ENGINEERING, BARKING, ESSEX, GB, vol. 66, no. 2, 1 January 2005 (2005-01-01), pages 211-217, XP004565551, ISSN: 0260-8774, DOI: 10.1016/J.JFOODENG.2004.03.008
- OTT G ET AL: "The Adjuvant MF59: A 10-Year Perspective", METHODS IN MOLECULAR MEDICINE,, vol. 42, 15 April 2000 (2000-04-15), pages 211-228, XP002501687, ISSN: 1543-1894

## Description

### Technical Field

The present invention relates to improved processes for the production of oil in water emulsions, in particular submicron oil in water emulsions comprising squalene.

### Background to the invention

The present invention relates to processes for the production of oil in water emulsions. Methods of manufacture are disclosed in Ott et al., 2000 (The Adjuvant MF59: A 10-year Perspective. Vaccine Adjuvants: Preparation methods and Research Protocols [Methods in Molecular medicine, Vol. 42, Chapter 12, p211-228], Ott et al., 1995 (MF59 - Design and Evaluation of a Safe and Potent Adjuvant for Human Vaccines: Vaccine Design, the Subunit and Adjuvant Approach [Pharmaceutical Biotechnology volume 6] eds. Powell & Newman, WO06/100110A1 and Lidgate et al., 1992 (Sterile Filtration of a Parenteral Emulsion. Pharmaceuticals Research 9(7): 860-863).

Oil in water emulsions are often used in vaccine/immunogenic compositions as adjuvants. As these emulsions are administered to humans it is necessary that the emulsions are sterile. Oil in water emulsions used as adjuvants are submicron emulsions and the oil droplets are sufficiently small to be sterile-filtered through 0.2µm filters. It is an object of the present invention to provide a process for the production of oil in water emulsions.

### Summary of the Invention

The present invention relates to a process for production of submicron oil in water emulsion, in particular submicron oil in water emulsions comprising squalene. In particular, the present invention relates to a process for the production of an oil in water emulsion comprising the step of a) introducing an oil phase into a mixing device by applying a positive pressure in the oil phase containing tank.

### Brief description of Figures

**Figure 1****:** Evolution of Z-average diameter of emulsion droplets with the number of passes and after filtration. Error bars are the standard deviations.
**Figure 2****:** Evolution of the polydispersity of emulsion droplets with the number of passes and after filtration. Error bars are the standard deviations.
**Figure** 3: Filterability, expressed as the capacity of the filter, of the emulsions after 3 passes, measured either on a 150 cm² membrane or on a 1300 cm² membrane. Error bars are the standard deviations.

### Detailed Description of the Invention

Oil in water emulsions are made by combining and mixing an oil phase (comprising one or more oils and optionally one or more surfactants) with an aqueous phase comprising a surfactant. The surfactant and microfluidisation allows a stable emulsion to be formed *i.e.* an emulsion that will not separate into oil and aqueous phases for at least 3 years.

The present inventors have demonstrated that by introducing an oil phase into a mixing device by applying positive pressure in a receptacle (for example a feeding tank) comprising the oil phase produces an oil in water emulsion with a reduced polydispersity, reduced droplet size and improved filterability compared to an oil in water emulsion in which the oil phase is introduced by, for example, a membrane pump.

Accordingly, the present invention provides a process for the production of an oil in water emulsion suitable for use the in the prevention and/or treatment of disease comprising the step of:
a. introducing an oil phase into a mixing device by applying a positive pressure in the oil phase containing tank, wherein the oil phase comprises squalene;
b. introducing an aqueous phase into said mixing device by applying a positive pressure in the aqueous phase containing tank;
c. mixing the oil and aqueous phases to form an oil in water emulsion.
d. subjecting the oil in water emulsion of step c) to high pressure homogenization to form a submicron oil in water emulsion;
e. pre-filtering the oil in water emulsion; and
f. filtering an oil in water emulsion filtered according to step e) through a sterile grade filter, wherein the oil and/or aqueous phases are introduced into the mixing device by adjusting the pressure to about 2 to 6 bars.

In order for any oil in water composition to be suitable for human administration, the oil phase must comprise a metabolisable oil (*i.e.* biodegradable). The oil may be any vegetable oil, fish oil, animal oil or synthetic oil, which is not toxic to the recipient and is capable of being transformed by metabolism. Nuts, seeds, and grains are common sources of vegetable oils. Synthetic oils are also suitable. Accordingly, oil in water emulsions of the invention comprise squalene (for example between about 4% and 6% [v/v]).

The oil phase may comprise one or more metabolisable oils. In a particular embodiment of the invention, the oil phase comprises a tocol and in a particular embodiment of the invention the oil phase comprises α-tocopherol.

The oil phase may further comprise a surfactant. Suitable surfactants are well known to the skilled person and include, but are not limited to polyoxyethylene sorbitan monooleate (TWEEN 80, POLYSORBATE 80), sorbitan triolate (SPAN 85), phosphatidylcholine (lecithin), polyoxyethylene (12) cetostearyl ether and octoxynol-9 (TRITON X-100). In a particular embodiment the oil phase comprises sorbitan triolate (SPAN 85).

The aqueous phase used in the processes of the invention may be water, for example water for injection (WFI). However, it is preferable that the aqueous phases of the invention comprise a buffer. Suitable buffers are well known to the person skilled in the art and include but are not limited to a phosphate buffer, citrate buffer, Tris buffer, succinate buffer, maleate buffer or borate buffer. The buffer may be selected from the group, phosphate buffered saline (PBS), modified PBS (PBS-mod) and citrate buffer. For example, the aqueous phase may comprise a buffer, that when mixed with the oil phase will provide a substantially isotonic oil in water emulsion.

In a particular embodiment of the invention, the aqueous phase comprises one or more surfactants as described herein. In a particular embodiment of the invention, the aqueous phase comprises the surfactant polyoxyethylene sorbitan monooleate (TWEEN 80, POLYSORBATE 80).

In a particular embodiment of the invention the introduction of the aqueous phase [step b)] and the oil phase [step a)] are performed substantially simultaneously.

In a further embodiment of the invention, the aqueous and oil phases as described herein are introduced at a ratio of about 90:10 or about 95:5 or about 97.5:2.5 or about 98.75:1.25 (percent v/v).

The term "mixing device" as used herein means a device suitable for mixing an oil phase and an aqueous phase to form an emulsion. In a particular embodiment of the invention the mixing device is a high shear mixing device. Suitable high shear mixing devices are known to the skilled person and include, but are not limited to a high-speed blade homogeniser, an inline homogeniser, a colloid mill or a sonolator. In a particular embodiment of the invention, the mixing device is a high-pressure homogeniser. Suitable high pressure homogenizers are known to the skilled person and include, but are not limited to a fixed geometry microfluidiser or to a variable geometry high pressure homogenizer.

Following mixing in step c) the oil in water emulsion may be a coarse oil in water emulsion if mixed in a high shear mixing device for example. In order to reduce the size of the oil droplets in the oil in water emulsion so that it is suitable for sterile filtration for example, the emulsion from step c) can be further processed in for example a high-pressure homogeniser.

Accordingly, the process of the invention comprises the step d) subjecting the oil in water emulsion of step c) to high pressure homogenization to form a submicron oil in water emulsion.

The skilled person can achieve the desired oil droplet size by varying the number of times the emulsion is passed through the high pressure homogeniser, as the oil droplet size will reduce after each cycle. Accordingly, in a one embodiment of the invention there is provided a process as described herein where in the emulsion is subjected to high pressure homogenisation [step f)] 1, 2, 3, 4, 5, 6, 7, 8 or more times.

In a further embodiment of the invention, the high pressure homogenization is performed at a pressure of between about 10000 and about 20000, about 12000 and about 18000, about 14000 and about 16000 or about 15000±1000 psi.

During high pressure homogenisation the temperature of the oil in water emulsion typically increases and thus in a one embodiment of the invention the oil in water emulsion is cooled to between about 15°C and about 30°C, about 16°C and about 29°C, about 17°C and about 28°C, about 16°C and about 27°C or between about 16 °C and about 28°C after the one or more, but at least the final time the emulsion is subjected to high pressure homogenisation.

By "sterile grade filter" it is meant a filter that produces a sterile effluent after being challenged by microorganisms at a challenge level of greater than or equal to 1x10⁷/cm² of effective filtration area. Sterile grade filters are well known to the person skilled in the art of the invention and have a pore size of about 0.2µm, and thus include filters with a pore size of about 0.22µm.

The filter used in the pre-filter (*i.e.* the filter used in step e) can have pores with the size ranging those of a sterile grade filter (i.e. about 0.2µm), to a pore size of about 2µm. In a particular embodiment the pore size of the pre-filter ranges from the size of those of a sterile grade filter sterile grade filter to the size of about 1µm. For example, the pore size of the filter used in step e) is about 2µm, about 2.5µm, about 1µm, about 0.9µm, about 0.8µm, about 0.9µm about 0.7µm, about 0.6µm, about 0.5µm, 0.45µm or sterile grade for example about 0.2µm, for example 0.22µm.

The membranes of the filter can be made from any suitable material known to the skilled person, for example, but not limited to cellulose acetate, polyethersulfone (PES), polyvinylidene fluoride (PVDF), polytetrafluoroethylene (PTFE). For example, one or more or all of the filter membranes of the present invention may comprise polyethersulfone, for example hydrophilic polyethersulfone.

In an embodiment of the invention, the submicron oil in water emulsions comprise between about 4% and about 6% (v/v) oil.

The processes of the invention produce oil in water emulsions in which the oil droplets are smaller, the polydispersity is reduced and/or the oil in water emulsion is more easily filtered compared to oil in water emulsions produced wherein the oil phase is introduced by means such as a membrane pump.

Accordingly, the present disclosure describes an oil in water emulsion produced by the processes as described herein.

The oil in water emulsions of the present invention may be useful as adjuvants and may be combined with more or more antigens to produce immunogenic compositions. Accordingly, the present disclosure describes an immunogenic composition comprising oil in water emulsions produced by the processes as described herein and an antigen.

Also described herein is a kit comprising: i) an oil in water emulsion produced by the processes as described herein; and (ii) an antigen.

Suitable antigens for use in immunogenic compositions or kits described herein include but are not limited to antigens derived from influenza virus. In particular, the influenza antigen may be subunit or a split influenza virus. The influenza antigen may be either egg-derived or in particular cell culture derived.

Oil in water emulsions of the invention may also comprise a tocol. Tocols are well known in the art and are described in EP0382271. In particular, the tocol is α-tocopherol or a derivative thereof such as alpha-tocopherol succinate (also known as vitamin E succinate).

Oil in water emulsions of the invention comprise one or more surfactants. Suitable surfactants are well known to the skilled person and include, but are not limited to polyoxyethylene sorbitan monooleate (TWEEN 80, POLYSORBATE 80), sorbitan triolate (SPAN 85), phosphatidylcholine (lecithin), polyoxyethylene (12) cetostearyl ether and octoxynol-9 (TRITON X-100). In particular, oil in water emulsions may comprise polyoxyethylene sorbitan monooleate (TWEEN 80, POLYSORBATE 80). Alternatively, oil in water emulsions may comprise polyoxyethylene sorbitan monooleate (TWEEN 80, POLYSORBATE 80) and a further surfactant, in particular sorbitan trioleate (SPAN 85).

In a particular embodiment of the invention the oil in water emulsion comprises a metabolisable oil (*e.g*. squalene), a tocol (*e.g*. α-tocopherol) and a surfactant (*e.g*. polyoxyethylene sorbitan monooleate [TWEEN 80, POLYSORBATE 80]).

In a further embodiment of the invention, oil in water emulsions of the invention comprise a metabolisable oil (*e.g*. squalene), a surfactant (*e.g*. polyoxyethylene sorbitan monooleate [TWEEN 80, POLYSORBATE 80]), and optionally a second surfactant (*e.g*. sorbitan trioleate [SPAN 85]).

In a further embodiment of the invention, oil in water emulsions of the invention comprise a metabolisable oil (*e.g*. squalene), a polyoxyethylene alkyl ether hydrophilic non-ionic surfactant (*e.g*. polyoxyethylene (12) cetostearyl ether) and a hydrophobic non-ionic surfactant (*e.g*. polyoxyethylene sorbitan monooleate [(TWEEN 80, POLYSORBATE 80)]), or sorbitan trioleate [SPAN 85]).

The oil in water emulsions and immunogenic compositions as described herein are suitable for use in medicine and specifically for the prevention and/or treatment of disease.

Accordingly, there is described an oil in water emulsion produced by the any processes described herein or an immunogenic composition as described herein for use in medicine.

There is further described an oil in water emulsion produced by the any processes described herein or the immunogenic composition as described herein for use in the prevention and/or treatment of disease in a mammal, in particular a human.

Further described is the use of an oil in water emulsion produced by the any processes described herein or the immunogenic composition as described herein in the manufacture of a medicament the prevention and/or treatment of disease in a mammal, in particular a human.

There is also described a method of treatment comprising the step of administering the oil in water emulsion produced by the any processes described herein or an immunogenic composition as described herein to a mammal, in particular a human.

The terms "comprising", "comprise" and "comprises" herein are intended by the inventors to be optionally substitutable with the terms "consisting of", "consist of" and "consists of", respectively, in every instance.

The term "about" in relation to a numerical value x means x ± 5% or 10%.

### Example

### Method

Aqueous phase was prepared in tank 2, by mixing water for injection, phosphate buffer saline and Tween. Oily phase was prepared in tank 1, by mixing tocopherol and squalene. Both phases were stirred until homogeneity was obtained. The whole installation was flushed with nitrogen in order to avoid tocopherol oxidation.

High pressure homogenizer was fed with a membrane pump. Both phases were fed together, with the required flow rate ratio. Both phases were first passed through a high shear homogenizer where a coarse emulsion was obtained. Then, in line with the high shear homogenizer, the product entered the high pressure homogenizer, were the fine emulsion was obtained.

At the outlet of the mixing device, the product was harvested in tank 3 (first pass). When tank 2 and 1 were empty, emulsion from tank 3 was directed towards the inlet of the mixing device and a second pass was performed. At the end of the second pass, the product is harvested in tank 2. When tank 3 was empty, the emulsion from tank 2 was directed towards the inlet of the mixing device and a third pass was performed. The emulsion was harvested in tank 3 and stored under nitrogen until filtration.

In order to assess the influence of the feeding method, processes were also performed with the whole installation submitted to a positive feeding pressure. In order to reach this, tanks were pressurized with nitrogen or air.

### Results

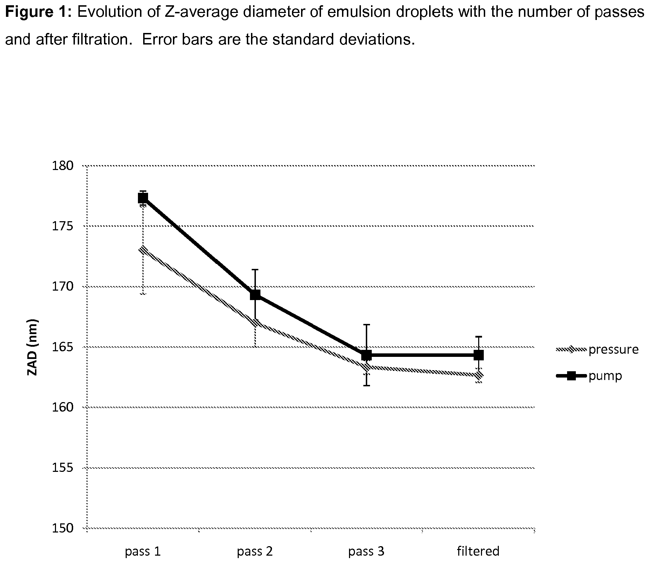

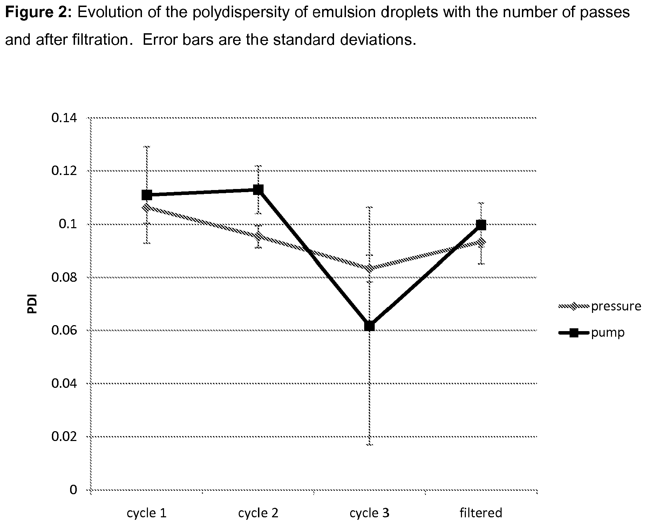

Size was measured by dynamic light scattering, a method based on light diffusion. Z-average diameter was smaller for processes using a membranes pump for the feeding compared to processes where the whole installation is submitted to positive pressure. This difference was larger at the beginning of the process (pass 1) but tended to vanish at the end of the process (filtrated product). Additionally, when feeding was performed with the whole system under positive pressure, the variability on size also decreased when passes were added. This is not the case when a membrane pump is used. See Figure 1. Polydispersity was in the same range, whatever the feeding method but, the reproducibility was much better when the process is performed with the whole installation submitted to a positive pressure. See Figure 2.

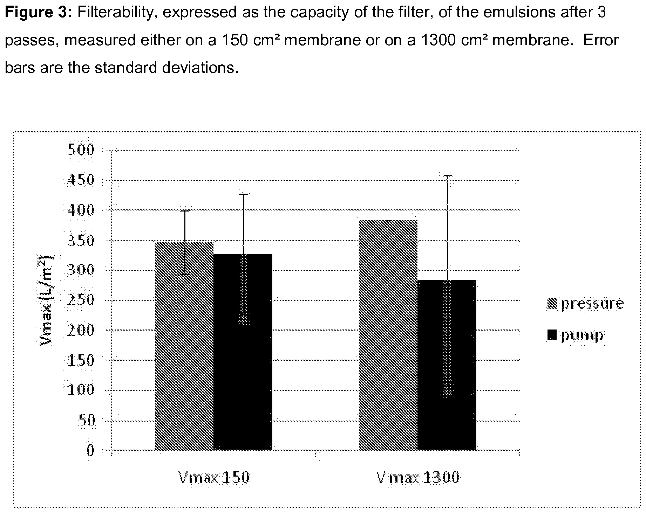

Filterability was increased, and most importantly the reproducibility was improved, when the process is performed with the whole installation submitted to a positive pressure compared to a process for which the feeding is done with a membrane pump. See Figure 3.

### Conclusion

Feeding the mixing device by submitting the whole installation to a positive pressure allows obtaining smaller droplet size and improved filterability. Moreover, it also improves the reproducibility of the process since standard deviation obtained on ZAD, PDI and filterability are smaller compared to a process for which the feeding is performed with a membrane pump.

## Claims

1. A process for the production of an oil in water emulsion suitable for use the prevention and/or treatment of disease comprising the step of:
a. introducing an oil phase into a mixing device by applying a positive pressure in the oil phase containing tank, wherein the oil phase comprises squalene;
b. introducing an aqueous phase into said mixing device by applying a positive pressure in the aqueous phase containing tank;
c. mixing the oil and aqueous phases to form an oil in water emulsion.
d. subjecting the oil in water emulsion of step c) to high pressure homogenization to form a submicron oil in water emulsion;
e. pre-filtering the oil in water emulsion; and
f. filtering an oil in water emulsion filtered according to step e) through a sterile grade filter.
wherein the oil and/or aqueous phases are introduced into the mixing device by adjusting the pressure to about 2 to 6 bars.

2. The process according to claim 1 wherein steps a) and b) are performed substantially simultaneously.

3. The process of any preceding claim wherein the aqueous and oil phases are introduced at a ratio of about 90:10, 95:5 or 98.75:1.25 (percent v/v).

4. The process of any preceding claim wherein the mixing device is a high shear homogenizer or a high pressure homogeniser.

5. The process of any one of claims 1 to 4 wherein step d) is performed 2, 3, 4, 5, or 6 times.

6. The process of any one of claims 1 to 5 wherein the high pressure homogenization is performed at between about 10000psi and about 20000psi, about 12000psi and about 18000psi, about 14000psi and about 16000psi or about 15000±1000 psi.

7. The process of any one of claims 1 to 6 wherein oil in water submicron emulsion is cooled to between 15°C and 30°C, 16°C and 29°C, 17°C and 28°C, 16°C and 27°C or between 16 °C and 28°C following each time step d) is performed.

8. The process of preceding claim wherein the oil in water emulsion comprises between 2% and 8% (v/v) oil.

9. The process of claim 8 wherein the submicron oil in water emulsion comprises between 4% and 6% (v/v) oil.

10. The process of any preceding claim wherein the oil in water emulsion comprises between 8 and 12% (v/v) oil.

11. The process of any preceding claim wherein the oil phase comprises a surfactant.

12. The process of claim 11 wherein the surfactant is sorbitan trioleate (SPAN 85).

13. The process of any preceding claim wherein the oil phase comprises a tocol, for example α-tocopherol.

14. The process of any one of claims 1 to 13 wherein the aqueous phase comprises an aqueous solution and a surfactant.

15. The process of claim 14 wherein the surfactant is polyoxyethylene sorbitan monooleate (TWEEN 80, POLYSORBATE 80).

## Patentansprüche

1. Verfahren zur Herstellung einer Öl-in-Wasser-Emulsion, die geeignet ist zur Verwendung in der Prävention und/oder der Behandlung von Krankheiten, umfassend die Schritte:
a. des Einbringens einer Ölphase in ein Mischvorrichtung durch Anlegen eines positiven Drucks an den die Ölphase enthaltenden Tank, wobei die Ölphase Squalen enthält;
b. des Einbringens einer wässrigen Phase in besagte Mischvorrichtung durch Anlegen eines positiven Drucks an den die wässrige Phase enthaltenden Tank;
c. des Mischens der Ölphase und der wässrigen Phase, um eine Öl-in-Wasser-Emulsion zu bilden;
d. des Unterwerfens der Öl-in-Wasser-Emulsion aus Schritt c) einer Hochdruck-Homogenisierung, um eine Öl-in-Wasser-Submikroemulsion zu bilden; und
e. des Vorfilterns der Öl-in-Wasser-Emulsion;
f. des Filterns einer Öl-in-Wasser-Emulsion, gefiltert gemäß Schritt e), durch einen sterilen Filter;
wobei die Ölphase und/oder die wässrige Phase in die Mischvorrichtung eingebracht werden, indem der Druck auf ungefähr 2 bis 6 bar eingestellt wird.

2. Verfahren gemäß Anspruch 1, wobei die Schritte a) und b) im Wesentlichen gleichzeitig durchgeführt werden.

3. Verfahren gemäß einem der vorangehenden Ansprüche, wobei die wässrige Phase und die Ölphase in einem Verhältnis von ungefähr 90:10, 95:5 oder 98,75:1,25 (Prozent v/v) eingebracht werden.

4. Verfahren gemäß einem der vorangehenden Ansprüche, wobei die Mischvorrichtung ein Hochscher-Homogenisator oder ein Hochdruck-Homogenisator ist.

5. Verfahren gemäß einem der Ansprüche 1 bis 4, wobei der Schritt d) 2-, 3-, 4-, 5- oder 6-mal durchgeführt wird.

6. Verfahren gemäß einem der Ansprüche 1 bis 5, wobei die Hochdruck-Homogenisierung zwischen ungefähr 10000 psi und ungefähr 20000 psi, ungefähr 12000 psi und ungefähr 18000 psi, ungefähr 14000 psi und ungefähr 16000 psi oder bei ungefähr 15000 ± 1000 psi durchgeführt wird.

7. Verfahren gemäß einem der Ansprüche 1 bis 6, wobei die Öl-in-Wasser-Submikroemulsion jedes Mal nachdem Schritt d) durchgeführt wurde auf zwischen 15 °C und 30 °C, 16 °C und 29 °C, 17 °C und 28 °C, 16 °C und 27 °C oder auf zwischen 16 °C und 28 °C gekühlt wird.

8. Verfahren gemäß dem vorangehenden Anspruch, wobei die Öl-in-Wasser-Emulsion zwischen 2% und 8% (v/v) Öl umfasst.

9. Verfahren gemäß Anspruch 8, wobei die Öl-in-Wasser-Submikroemulsion zwischen 4% und 6% (v/v) Öl umfasst.

10. Verfahren gemäß einem der vorangehenden Ansprüche, wobei die Öl-in-Wasser-Emulsion zwischen 8% und 12% (v/v) Öl umfasst.

11. Verfahren gemäß einem der vorangehenden Ansprüche, wobei die Ölphase ein Tensid umfasst.

12. Verfahren gemäß Anspruch 11, wobei das Tensid Sorbitantrioleat (SPAN 85) ist.

13. Verfahren gemäß einem der vorangehenden Ansprüche, wobei die Ölphase ein Tocol umfasst, beispielsweise α-Tocopherol ist.

14. Verfahren gemäß einem der Ansprüche 1 bis 13, wobei die wässrige Phase eine wässrige Lösung und ein Tensid umfasst.

15. Verfahren gemäß Anspruch 14, wobei das Tensid Polyoxyethylen-Sorbitanmonooleat (TWEEN 80, POLYSORBAT 80) ist.

## Revendications

1. Procédé pour la production d'une émulsion huile dans eau appropriée pour être utilisée dans la prévention et/ou le traitement d'une maladie comprenant l'étape consistant à :
a. introduire une phase huileuse dans un dispositif de mélange en appliquant une pression positive dans le réservoir contenant la phase huileuse, dans lequel la phase huileuse comprend du squalène ;
b. introduire une phase aqueuse dans ledit dispositif de mélange en appliquant une pression positive dans le réservoir contenant la phase aqueuse ;
c. mélanger les phases huileuse et aqueuse pour former une émulsion huile dans eau ;
d. soumettre l'émulsion huile dans eau de l'étape c) à une homogénéisation à haute pression pour former une émulsion huile dans eau sous-micronique ;
e. préfiltrer l'émulsion huile dans eau ; et
f. filtrer une émulsion huile dans eau filtrée selon l'étape e) au moyen d'un filtre de grade stérilisant,
dans lequel les phases huileuse et/ou aqueuse sont introduites dans le dispositif de mélange en ajustant la pression à environ 2 à 6 bars.

2. Procédé selon la revendication 1, dans lequel les étapes a) et b) sont réalisées sensiblement en même temps.

3. Procédé selon l'une quelconque des revendications précédentes, dans lequel les phases huileuse et aqueuse sont introduites à un rapport d'environ 90:10, 95:5 ou 98,75:1,25 (v/v pour cent).

4. Procédé selon l'une quelconque des revendications précédentes, dans lequel le dispositif de mélange est un homogénéiseur à cisaillement élevé ou un homogénéiseur à haute pression.

5. Procédé selon l'une quelconque des revendications 1 à 4, dans lequel l'étape d) est réalisée 2, 3, 4, 5 ou 6 fois.

6. Procédé selon l'une quelconque des revendications 1 à 5, dans lequel l'homogénéisation à haute pression est réalisée entre environ 10 000 psi et environ 20 000 psi, entre environ 12 000 psi et environ 18 000 psi, entre environ 14 000 psi et environ 16 000 psi ou à environ 15 000 ± 1 000 psi.

7. Procédé selon l'une quelconque des revendications 1 à 6, dans lequel une émulsion huile dans eau sous-micronique est refroidie à entre 15 °C et 30 °C, entre 16 °C et 29 °C, entre 17 °C et 28 °C, entre 16 °C et 27 °C ou entre 16 °C et 28 °C chaque fois après que l'étape d) est réalisée.

8. Procédé selon l'une quelconque des revendications précédentes, dans lequel l'émulsion huile dans eau comprend entre 2 % et 8 % (v/v) d'huile.

9. Procédé selon la revendication 8, dans lequel l'émulsion huile dans eau sous-micronique comprend entre 4 % et 6 % (v/v) d'huile.

10. Procédé selon l'une quelconque des revendications précédentes, dans lequel l'émulsion huile dans eau comprend entre 8 et 12 % (v/v) d'huile.

11. Procédé selon l'une quelconque des revendications précédentes, dans lequel la phase huileuse comprend un tensioactif.

12. Procédé selon la revendication 11, dans lequel le tensioactif est le trioléate de sorbitane (SPAN 85).

13. Procédé selon l'une quelconque des revendications précédentes, dans lequel la phase huileuse comprend un tocol, par exemple le α-tocophérol.

14. Procédé selon l'une quelconque des revendications 1 à 13, dans lequel la phase aqueuse comprend une solution aqueuse et un tensioactif.

15. Procédé selon la revendication 14, dans lequel le tensioactif est le monooléate de polyoxyéthylène sorbitane (TWEEN 80, POLYSORBATE 80).
